# EUROPEAN PATENT APPLICATION

(11) **EP 2 003 582 A2**
(43) Date of publication of application: **17.12.2008**
(21) Application number: 08157869.2
(22) Date of filing: 09.06.2008
(51) Int. Cl.: G06F 19/00

(54) **Systems and methods of telemonitoring**

(30) Priority: 12.06.2007 US 761742
(71) Applicant: Honeywell International Inc., Morristown NJ 07960 (US)
(72) Inventor: Budish, Christopher D., Menomonee Falls, WI 53051 (US); Rayfield, Melissa A, Morristown, NJ 07962 (US); Schwabe, Jackie A., Milwaukee, WI 53225 (US)
(74) Representative: Haley, Stephen

(57) **Abstract**

A monitoring system can be used with a passive exercise-type machine, or a touch pad. An exercise schedule can be transferred to the system for use with the passive exercise-type machine. In an alternate embodiment, an exercise program can be presented for use with the touch pad.

## Description

The invention pertains to systems and methods for monitoring patients from remote locations. More particularly, the invention pertains to such systems and methods where patient related information can be transferred to/from a displaced monitoring site to reduce needs for medical professionals to physically visit patients.

There is a shortage of physical therapists through out the world. This shortage is going to continue to grow and needs to be addressed.

There are approximately 267,000 knee replacements a year. Other knee related issues that require rehabilitation include total knee replacements, total knee reconstructions, ligament and tendon repairs, arthroscopic surgery, articular features/fractures, articular cartilage repair, and meniscus repairs. Each of these issues are treated post operatively using a continuous passive movement machine.

Currently the machines are dropped off at the patient's home and they are given brief instructions before surgery on how to use the machine. These machines are used to increase a patient's range of motion over a specific period of time. The degrees that the patient can bend their knee and the duration that they are able to use the machine are all necessary to assist the patient in recovering. Other types of joint therapies such as shoulders or ankles might use similar types of exercise machines.

There is also a large group of individuals that have had a stroke, that are autistic, or that have other neurological issues. These issues are resolved with therapy to increase motor skills.

There is an ongoing need for a solution that enables more patients to be cared for with fewer physical therapists. Such a solution would make it possible for patients to continue to get the care they need in spite of this shortage of therapists.

### In the Drawings;

Fig. 1 is a block diagram illustrating one embodiment of the present invention;

Figs. 2A-2C taken together illustrate a plurality of patient information/control screens displayable at the system of FIG. 1;

Fig. 3 illustrates exemplary contact pads usable with another embodiment of the invention; and

Fig. 4 is a block diagram of another system in accordance with the invention.

While embodiments of this invention can take many different forms, specific embodiments thereof are shown in the drawings and will be described herein in detail with the understanding that the present disclosure is to be considered as an exemplification of the principles of the invention, as well as the best mode of practicing same, and is not intended to limit the invention to the specific embodiment illustrated.

Embodiments of the invention incorporate continuous Passive Motion (CPM) machines used to rehabilitate physical therapy (PT) knee surgery patients to wirelessly transfer degrees, duration, and descriptions of their pain to displaced monitoring sites. Such embodiments can be expected to result in a need for fewer nurses, fewer patient visits, and fewer reinjuries by monitoring compliance remotely on a regular basis. Fewer in-patient visits enables both more PT to be provided as well as fewer visits and more time for therapists to assist other patients.

In one aspect of the invention CPM machines could be linked to a telemonitoring system that sends the data to the PTs to track progress while reducing visits. A wireless interface modem or pager could be installed in the CPM machine to enable it to send information to a central monitoring system on a scheduled basis. Embodiments of the invention can be expected to decrease the amount of time for postoperative care by being able to work with the patient to be sure he/she is doing the exercises correctly.

In accordance with the invention, passive exercise machines can be implemented with a graphical users interface and a wireless interface. They can be configurable remotely from a Central Monitoring Station. A display can be provided which permits entry of as many repetitions as is needed per day based on a treatment plan. Such machines are beneficial for those patients that might have trouble entering the treatment plan.

In one aspect, the interface can be loaded at the time of post-surgery discharge with discharge instructions, the configuration needed on the monitor, and the limits that need to be set for repetitions, duration, degrees, schedules, etc. Compliance can be validated by checking how the patient is progressing (trending) relative to degrees, repetitions, and schedules. The success factors include shorter treatment duration, a decrease in the cost to insurance companies; less time needed to be spent by nurses or therapists as fewer patients' visits are needed and less reinjury. Embodiments of the invention can be used for post-op back surgery, post-op knee surgery, post-op shoulder surgery treatments. Additionally, the location of the equipment can be tracked.

In another aspect of the invention; a care plan entry screen could include dates for start and end of the time on the machine, the degree target, the frequency per day target, limits that alert if the target degree is exceeded, or not reached in a specific time period. If the number of repetitions or frequency per day is exceeded or not reached during a specific time frame, an alert can be sent to the Central Monitoring Station. The care plan can also be changed remotely or physically at the device.

In the event that maintenance is needed a request can be sent via the wireless interface. A user can use a help button on the interface to contact a person at the central monitoring location. It could also provide additional independence for the person in the home.

In yet another aspect of the invention, the patient via the user interface, can enter information as to his/her condition. These can include responses to subjective questions like: is your leg, knee, etc. swollen? "Yes" or "no" buttons on the device can be used as inputs. The standard pain scale can be displayed. The patient can push a button to indicate the amount of pain he/she is experiencing at the current setting. This may assist the Central Monitoring Station personnel in identifying a potential issue.

Automated voice instructions can be provided on how to set up the exercise machine. The instructions are verbal and can be presented to the patient in his/her primary language. The therapist can setup changes in the care plan remotely as well.

Embodiments of the invention enable a patient to improve faster and spend less time going for PT where he/she has to travel with a potential to re-injure themselves due to limited mobility. This feature should also reduce the cost of treatment.

In yet another aspect, there is a multi-patient component. For example, multiple treatment programs can be stored. An assisted nursing facility could have more than one patient using a device and save the cost of needing a device for each patient that would need it. There are 267,000 knee replacements each year. In addition, there are total knee replacements, all reconstruction of the knee, and ligaments and tendons repairs, also arthoscopic surgery, stable intra-articular features/fractures, articular cartilage repair, meniscus repair, joint manipulation, and other applications where embodiments of the invention can be used to provide therapy.

In yet another aspect, alternate embodiments of the invention can receive inputs from an exercise sequence when a person uses a sensor or a mat. Such information could be captured and put into a packet of data to be sent to their caregiver, for example, a neurologist. It could used to evaluate how they are or are not progressing.

They could be on a schedule of when to the play the game. A game could be setup to test specific neurological functions - like how long it takes before they do what they are shown on the screen, or how fast they are able to go from one item to another, or if they are able to see an act on specific color first and/or if their transitions between on color and another are faster or slower than other combinations. Voice activated questions are available that can ask the patient to perform an action on the mat. This will test their auditory skills or their ability to hear and retain and act on information. All of this information can be housed and tracked and used for trending to evaluate compliance and success of the patient's care plan.

Embodiments of the invention can use sensors to track the number of times, within a specific duration time; an individual touches a sensor on a color coded mat. A disclosed embodiment of the invention could be remotely programmed at a central station. The system would tell the patient when it is time to start the session. Performance, or feedback, information can then automatically sent to a doctor or physical therapist to indicate the trends and the progress the person is making. If they are not making progress then it might alert the PT to schedule a visit.

Embodiments of the invention can store and keep track of patient progression and changes, such as, increases in motor skills, with fewer in-person visits by a therapist. Trends can also be observed and extracted from the daily data to allow potential problems to be detected earlier. Embodiments of the invention fill a gap between resources and needs by using telemonitoring to extend available resources.

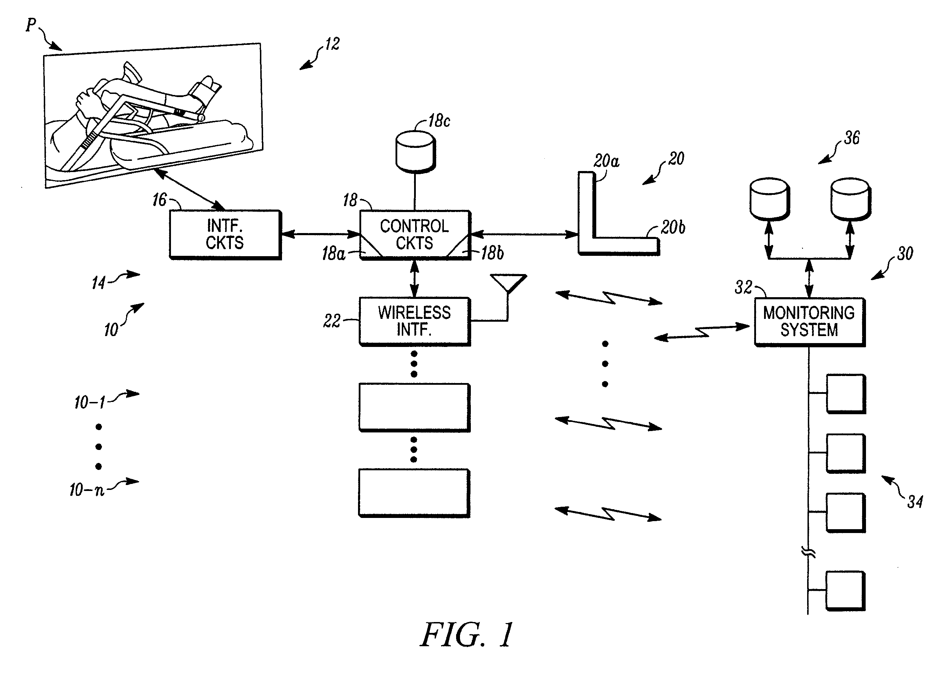

FIG. 1 illustrates system 10 which embodies the present invention. System 10 includes a continuous passive motion exercise unit 12, for example, which might be used to treat a patient P subsequent to knee surgery of various types. Other types of passive motion devices can be used for ankle, shoulder, elbow, wrist or back surgery.

Device or unit 12 can be coupled to a local control element 14 via interface or circuits 16. Control circuits 18, coupled to the interface circuits 16, can provide drive or control signals to the unit 12 on a timed basis. Control circuits 18 can be implemented at least in part with a programmable processor 18a, in combination with executable control instructions 18b.

One or more treatment schedules can be stored on disc drive 18c, accessible to control circuits 18. A human interface device 20 can be coupled to control circuit 18. Device 20 can include a multi-dimensional, graphical display device 20a as well as an input device such as a keyboard 20b. Control circuits 18 can present via device 20 a plurality of different screens, and/or receive inputs or information relative thereto, via a graphical users interface, from a patient, therapist or other patient representative. Audible, verbal, outputs can also be generated by circuits 18 and output via one or more speakers in one of units 18, 20.

System 14 can also include a wireless interface 22 coupled to the control circuits 18 for purposes of transmitting data to, receiving data, and instructions from a displaced monitoring system 30. System 30 can include one or more of control computers 32, a plurality of display devices 34 which could be used by medical professionals such as nurses, physical therapists, occupational therapists and alike all without limitation. Patient records and schedules particularly for exercise and for other treatment can be maintained on disc drives and databases 36.

As desired, treatment scheduled can be downloaded from system 30 to the unit 14 for purposes of establishing a treatment program. Alternately, such information can be entered locally via device 20.

It will also be understood that a plurality of devices such as 10-1, 10-2. 10-n comparable to system 10 could be dispersed geographically in an area and in intermittent communication as necessary with the system 30. In such instances, the various systems 10-i each could be set up either remotely or locally with a schedule customized to the needs of a particular individual's treatment plan. Each of the systems 10-i would include a cpm unit such as unit 12, or depending on the procedure, a different type of cpm unit appropriate for the type of joint surgery from which the patient is recovering.

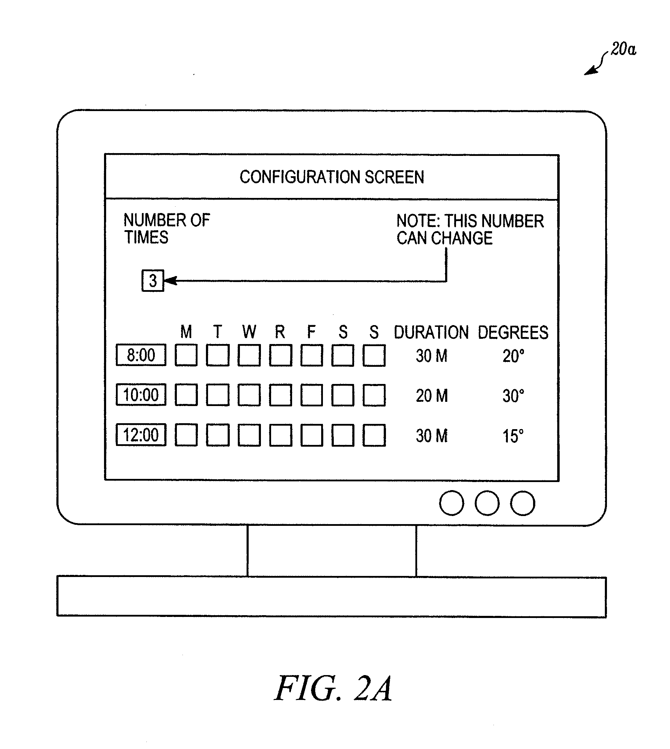

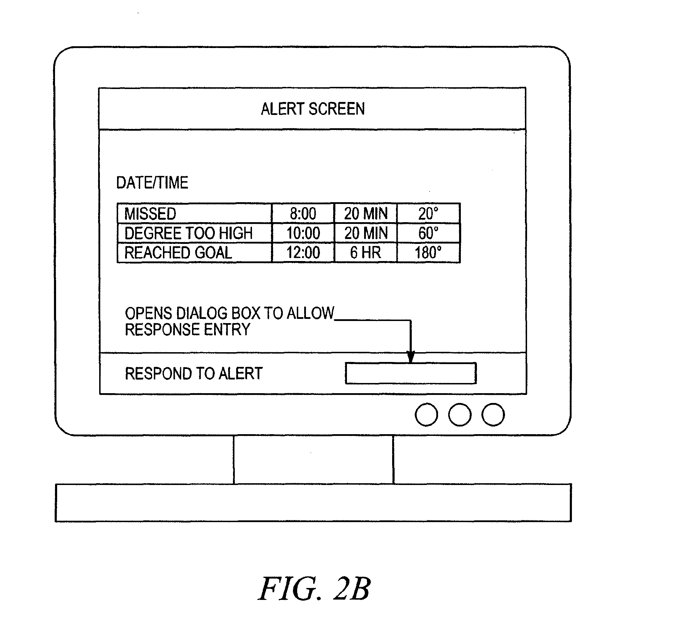

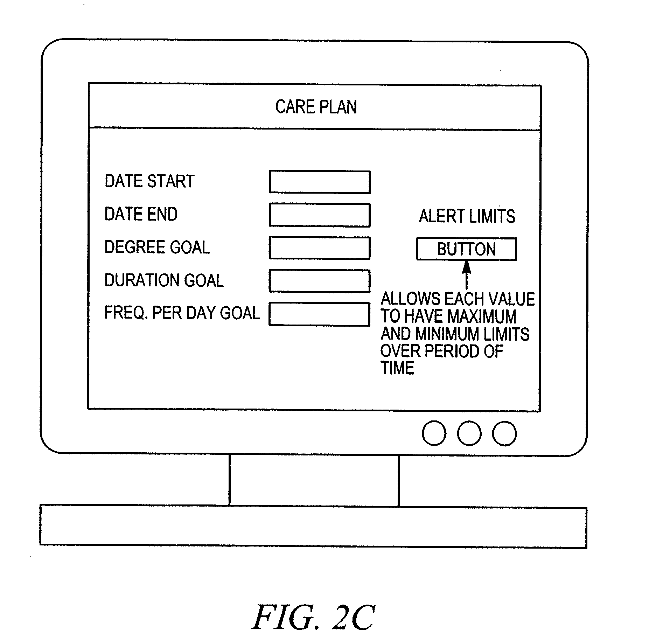

FIGS. 2A-2C illustrate various screens of the type presentable by the system 10 on the display unit 20a pertaining to configuring the unit 12 with a schedule for a particular patient, FIG. 2A; an alert screen FIG. 2B which provides information as to day/time of treatment, duration and degrees of motion, FIG. 2B; and a display of a care plan, FIG. 2C, which contains information pertaining to starting and ending of the exercise therapy, goals, duration and other related information.

It will be understood that other screens could be developed and presented on the display unit 20a. For example, a comfort scale screen which reflects patient discomfort or pain in conjunction with degrees of motion, numbers of times per day of exercise as well as duration can be displayed for the individual to enter comfort related feedback information usable by the therapists or nurses at the system 30.

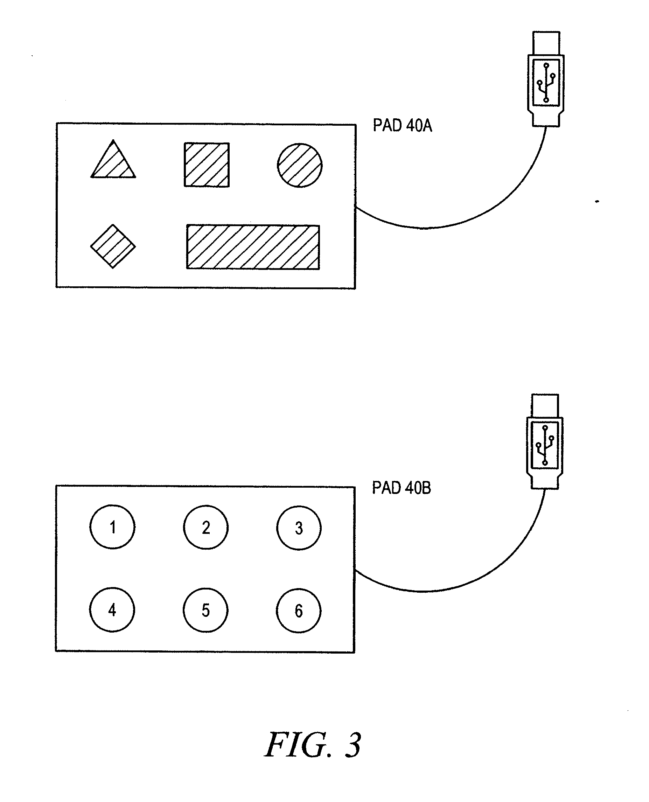

Fig. 3 illustrates various mat or pad configurations such as 40a, 40b usable with another embodiment of the present invention. Pads such as 40a, 40b can be printed with a variety of shapes, numbers, colors and the like all without limitation. Such pads, or mats, sometimes marketed as "dance pads" provide location information on a real-time basis as an individual on the pad moves about.

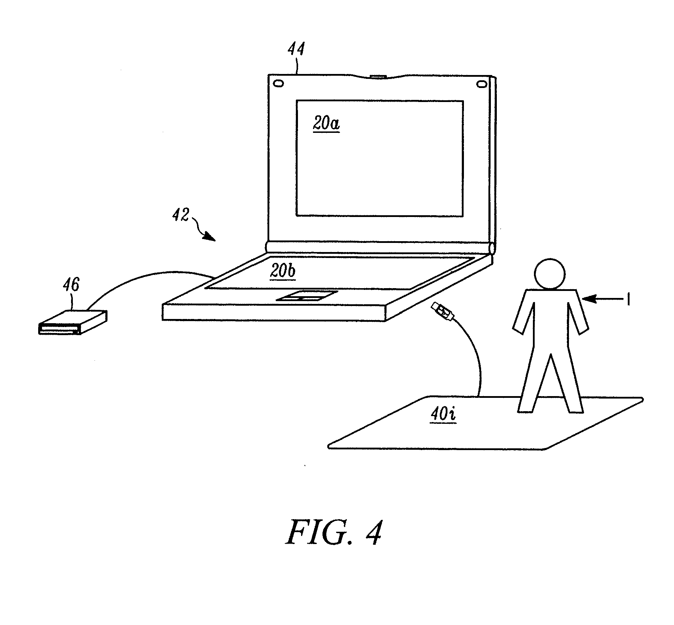

Pads such as 40a, 40b can be coupled to a respective computer 44, best seen in Fig. 4. A variety of connectors and connection protocols are available. For example, the pad 40i could be coupled to a USB port on the computer 44.

Systems such as the system 42 can be equipped with a CD/DVD player 46. System 42 could also incorporate a wireless interface such as the wireless interface 22 discussed previously.

One or more activity programs can be stored on a CD or DVD and played on the player, or drive 46, or, stored in memory of the computer 44. A prestored exercise program can be activated and executed on the computer 44 and used in conjunction with audio from the player 46 and the activity mat 40i.

A game can be carried on with an individual I who is moving about on the mat 40i and who responds to both visual images on the display 20a as well as verbal or other audio outputs, which might be generated off the player 46.

One or more of the programs stored on the computer or processor 44 can be a scheduling program. The stored games could be organized and arranged to test specific neurological functions and responses. Information as to the ongoing progress of a respective game being carried out by an individual I can be stored and forwarded by computer 44 by a wireless interface 22 to a monitoring system 30.

Medical professionals including therapists, neurologists and the like can evaluate the incoming performance information which can include responses to screen images, response times as to how fast the individual goes from one item to another, responses to specific colors and the like all without limitation.

The system 42 can pose verbal questions or prompts that ask the individual I to perform an action on the activity pad 40i. The response of the individual is an indication of the individual's auditory skills and/or his or her ability to hear, retain and act on the requested information. All such feedback can be coupled via computer 44 to the system 30 for evaluation by the appropriate medical professional.

From the foregoing, it will be observed that numerous variations and modifications may be effected without departing from the spirit and scope of the invention. It is to be understood that no limitation with respect to the specific apparatus illustrated herein is intended or should be inferred. It is, of course, intended to cover by the appended claims all such modifications as fallen within the scope of the claims.

## Claims

1. A monitoring system for a continuous passive motion exercise unit comprising:
a control panel that can be used to establish an exercise schedule for a person recovering from a surgical procedure;
control circuits coupled to the panel that can receive and store an exercise schedule; and
a wireless communications interface, coupled to the control circuits that transmits exercise related information for the person to a displaced site;
where the control circuits include an interface to a continuous passive motion exercise unit.

2. A system as in claim 1 where the control panel includes a multi-dimensional display unit, and the control circuits present a graphical user's interface on the display unit.

3. A system as in claim 2 which includes circuitry to download an exercise schedule, via the wireless communications interface, from the displaced site.

4. A system as in claim 2 which includes voice output circuitry coupled to the control circuits which provides verbal feedback for the person.

5. A system as in claim 4 where the control panel includes at least one manually operable input that provides exercise related feedback.

6. A system as in claim 5 where the control circuits forward the exercise related feedback to the displaced site via the communications interface.

7. A monitoring system for a touch pad comprising:
control circuits;
a graphical display unit coupled to the control circuits including graphical user's interface software;
a first interface, coupled to the control circuits, to transfer and receive information to and from a displaced monitoring location;
a second interface, coupled to the control circuits and an input/output port, the port can be coupled to an exercise-type touch pad, the second interface receives contact indicating signals, via the port, indicative of activities at the touch pad, the second interface transfers representations of the signals to the control circuits.

8. A system as in claim 7 which includes exercise presentation circuitry that visually presents a sequence of exercises for use with the touch pad.

9. A system as in claim 8 where the presentation circuitry releasibly receives an exercise sequence carrying medium.

10. A system as in claim 9 where the medium is one of an optical medium or a magnetic medium and the system includes a display unit and a graphical user's interface, coupled to the common unit, that presents exercise indicia associated with a selected person.

11. A system as in claim 7 where the first interface transmits wireless signals to the displaced monitoring location.

12. A system as in claim 7 where the control circuits include at least one programmable processor and associated control software.

13. A system as in claim 12 which includes a unit that stores at least one exercise schedule.

14. A system as in claim 12 where the control software, when executed by the processor, periodically forwards, via the communications interface, exercise indicia related to the person.

15. A system as in claim 1 which includes, at the displaced site, a common unit that stores exercise indicia related to a plurality of persons.
